(19) Europäisches Patentamt
European Patent Office
Office européen des brevets

(11) Veröffentlichungsnummer: **0 534 316 A1**

# (12) EUROPÄISCHE PATENTANMELDUNG

(21) Anmeldenummer: **92115925.7**

(22) Anmeldetag: **17.09.92**

(51) Int. Cl.5: **A61K 31/785**, C08G 73/04, B01J 20/32

(30) Priorität: **21.09.91 DE 4131506**

(43) Veröffentlichungstag der Anmeldung:
**31.03.93 Patentblatt 93/13**

(84) Benannte Vertragsstaaten:
**AT BE CH DE DK ES FR GB GR IT LI LU MC NL PT SE**

(71) Anmelder: **HOECHST AKTIENGESELLSCHAFT**
**Postfach 80 03 20**
**W-6230 Frankfurt am Main 80(DE)**

(72) Erfinder: **Müllner, Stefan, Dr.**
**Freidrich-Ebert-Strasse 43**
**W.6203 Hochheim(DE)**

## (54) Verwendung von alkylierten Polyethyleniminderivaten zur Anreicherung von Gallensäuren.

(57) Es wird die Verwendung von unvernetzten cycloalkylierten Polyethyleniminen zur Anreicherung von Gallensäuren und deren Derivaten aus biologischen Flüssigkeiten oder Extrakten beschrieben.

EP 0 534 316 A1

Die Erfindung betrifft die Verwendung von alkylierten Polyethyleniminderivaten.

Unlösliche, basische, vernetzte Polymere werden seit geraumer Zeit zur Bindung von Gallensäure verwendet und aufgrund dieser Eigenschaften therapeutisch genutzt. Als Therapieobjekt werden die chologene Diarrhoe (z.B. nach Ileumresektion) und erhöhter Cholesterin-Blutspiegel kausal behandelt. Im letzteren Fall handelt es sich um den Eingriff in den enterohepatischen Kreislauf, wobei anstelle des aus dem Kreislauf genommenen Gallensäureanteils die entsprechende Neusynthese aus Cholesterin in der Leber provoziert wird. Zur Deckung des Cholesterinbedarts in der Leber wird auf das zirkulierende LDL (Low Density Lipoprotein)-Cholesterin zurückgegriffen, wobei die hepatischen LDL-Rezeptoren in vermehrter Anzahl zur Wirkung kommen. Die so erfolgte Beschleunigung des LDL-Katabolismus wirkt sich durch die Herabsetzung des atherogenen Cholesterinanteils im Blut aus.

Die als Arzneimittel in der Verwendung befindlichen Ionenaustauscher besitzen als aktive Gruppen entweder quartäre Ammoniumgruppen (wie Colestyramin) oder sekundäre bzw. tertiäre Aminogruppen (wie Colestipol). Die Tagesdosis an Colestyramin beträgt zweckmäßigerweise 12 - 24 g, als Tageshöchstdosis werden 32 g empfohlen. 15 - 30 g ist die empfohlene tägliche Colestipol-Dosis. Geschmack, Geruch und hohe Dosierung erschweren die Patienten-Compliance. Die Nebenwirkungen gehen auf mangelnde Selektivität (z.B. Avitaminosen) zurück, die auch bei der Dosierung simultan gegebener Medikamente berücksichtigt werden müssen, aber auch auf Gallensäureverarmung, die verschiedene gastrointestinale Störungen (Obstipation, Steatorrhoe) unterschiedlichen Grades hervorrufen. Für beide Präparate wurde eine therapeutische Bedeutung durch Kombination mit anderen hypolipidämisch wirkenden Pharmaka wie Fibrate, HMG-CoA-Reduktase-Inhibitoren, Probucol (vgl. z.B. M.N. CAYEN, Pharmac. Ther. 29, 187 (1985) und 8th International Symposium on Atherosclerosis, Rome, Oct. 9-13, 1988, Abstracts S. 544, 608, 710) beschrieben, wobei die erzielten Effekte auch die Therapie von schweren Hyperlipidämien ermöglichen. Deshalb erscheint es bedeutungsvoll, bei dem gegebenen Wirkprinzip, geeignete Stoffe ohne die Nachteile der gegenwärtig verwendeten Präparate zu finden.

Folgende Merkmale der genannten Präparate und insbesondere von Colestipol sind als verbesserungswürdig anzusehen:

1. Die hohen Tagesdosen, die zurückzuführen sind auf eine relativ geringe Bindungsrate bei neutralem pH in isotonem Medium und der (teilweisen) Wiederfreisetzung der adsorbierten Gallensäure.
2. Die qualitative Verschiebung der Gallensäurezusammensetzung der Galle mit abnehmender Tendenz für Chenodesoxycholsäure und die damit verbundene zunehmende Gefahr für Cholelithiasis.
3. Das Fehlen einer dämpfenden Wirkung auf den Cholesterinstoffwechsel der Darmbakterien.
4. Die zu hohe Bindungsrate von Vitaminen und Pharmaka macht einen Substitutionsbedarf an diesen Stoffen und Blutspiegelkontrollen eventuell notwendig.
5. Eine weitere Verbesserung kann in der Darreichungsform erzielt werden.

Die Behebung der aufgelisteten Mängel gelingt überraschenderweise durch die Anwendung hochmolekularer alkylierter Polyethylenimine. Die nicht resorbierbaren Makromoleküle entfalten ihre Wirkung sowohl in löslicher Form als auch pH-abhängig in unlöslicher Form, entsprechend der unvernetzten Struktur, als auch in unlöslichem Zustand als vernetzte Polymere.

Vernetzte Polyethylenimine werden in der US-Patentschrift 3 332 841 beschrieben. Die Vernetzung erfolgt u.a. über Alkylengruppen mit 2 bis 8 Kohlenstoffatomen, das Molekulargewicht der Ausgangspolymeren liegt zwischen 800 und 100 000. Zur Behandlung von vorübergehender Hyperacidität des Magens werden 0,25 bis 5 g pro Dosierungseinheit verabreicht. Es wird weder die Bindung von Gallensäure noch eine damit verbundene lipidsenkende Aktivität der vernetzten Polyethylenimine beschrieben, denn ohne Alkylierung besitzen die Polyethylenimine gegenüber den Gallensäuren je nach Typ keine oder nur unbedeutende Bindungskapazität. Durch die große potentielle Ladungsdichte kann durch die Alkylierung für die ausreichende Bindungskapazität und durch die Wahl der Substituenten von entsprechend hydrophil/hydrophobem Charakter für die Affinität und Bindungsspezifität gesorgt werden.

In der DE-A-39 01 527 (entsprechend US-Patentanmeldung Nr. 07/466,923 bzw. Nr. 07/762,177 und EP-A-0 379 161) werden unvernetzte und vernetzte alkylierte Polyethyleniminederivate beschrieben, bei denen das Grundpolymer Molekulargewichte von 10 000 bis 10 000 000 aufweist und die Alkylierungsmittel der allgemeinen Formel R-X entsprechen, wobei X ein Halogen, Pseudohalogen oder halogen-ähnliche Verbindung ist, R ein geradkettiger oder verzweigter $C_1$-$C_{30}$-Alkylrest ist, und im Falle der vernetzten alkylierten Polyethylenimine das Vernetzungsmittel ein alpha, omega-Dihalogenalkan mit 2-10 Kohlenstoffatomen oder ein höher funktionelles Halogenalkan mit 2-10 Kohlenstoffatomen ist.

In der gleichzeitig eingereichten deutschen Patentanmeldung P 41 31 507.3 werden unvernetzte cycloalkylierte Polyethylenimine eines Molekulargewichtes von 10 000 bis 10 000 000, herstellbar aus einem Ausgangspolyethylenimin eines Molekulargewichts von 10 000 bis 10 000 000 und einem Cycloalkylierungsmittel der Formel I

R-X (1)

worin

R ein Cycloalkylrest mit 5-30 Kohlenstoffatomen der monocyclisch, bicyclisch, tricyclisch, polycyclisch und/oder verbrückt sein kann und

X Chlor, Brom, Jod, $CH_3$-$SO_2$-O- oder

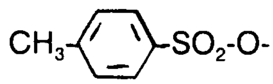

bedeutet, beschrieben.

Diese Verbindungen wurden nunmehr zur Anreicherung von Gallensäure aus biologischen Flüssigkeiten und Extrakten verwendet.

Insbesondere bevorzugt sind unvernetzte Polyethylenimine, in denen R Cyclopentyl, Cyclohexyl, Cycloheptyl, Cyclooctyl, Cyclononyl, Cyclodecyl, bicyclische Systeme wie Decalyl, Hydrindanyl, verbrückte Systeme wie Norbornyl, oder polycyclische Systeme wie z.B. Cyclopentanoperhydrophenanthren und davon abgeleitete Ringsysteme oder Derivate bedeutet.

Vorzugsweise werden Polyethylenimine mit einem Molekulargewicht über 100 000 eingesetzt.

In den Alkylierungsmitteln R-X ist X vorzugsweise Chlor oder Brom.

Das Verhältnis des eingesetzten Alkylierungsmittels zu den Aminogruppen des Polyethylenimins beträgt 0.2 : 1 bis 5 : 1, vorzugsweise 0.5 : 1 bis 2 : 1.

Durch die Reaktion mit Alkylierungsmittel wird ein Teil der sekundären Aminogruppen der Kette in tertiäre und quartäre Strukturen überführt. Die Bildung von tertiären Aminogruppen wird bevorzugt.

Zu den Gallensäuren zählen natürliche Gallensäuren, Derivate der natürlichen Gallensäuren und deren Alkali- bzw. Erdalkalisalze. Insbesondere zählen hierzu: Glycocholat, Taurocholat, Cholat, Cholsäure, Desoxy-, Chenodesoxy-, Ursodesoxy- und Lithocholsäure sowie deren Konjugate mit Glycin oder Taurin. Zusätzlich kann auch Cholesterin adsorbiert werden.

Unter Serum wird insbesondere Human- oder Animal-Serum verstanden.

Die erfindungsgemäß verwendeten alkylierten Polyethylenimine adsorbieren körpereigene Säuren, insbesondere Gallensäuren. Aufgrund dieser Eigenschaften sind sie in der Lage, erhöhte Cholesterinspiegel zu senken. Erhöhte Konzentrationen an Cholesterin, aber auch an Cholsäure, Chenodesoxycholsäure, Desoxycholsäure und Lithocholsäure treten u.a. bei folgenden Erkrankungen auf: Primäre biliäre Zirrhose (PBC), Cholestase und biliäre Atresie. Mit den alkylierten Polyethleniminen können in einem analytischen Verfahren die Konzentrationen der Gallensäuren wieder normalisiert werden.

Das erfindungsgemäße analytische Verfahren kann insbesondere in einer Ionenaustauschchromatographie bestehen, bei dem die an die Festphase gebundenen alkylierten Polyethylenimine zur Anreicherung der Gallensäure dienen.

Die beschriebene Verwendung dieser Verbindungen in analytischen Verfahren ist insbesondere deshalb sehr wichtig, weil der Nachweis von Gallensäure im Serum im allgemeinen außerordentlich schwierig ist.

Nach Leberpassage verbleibt nur noch ein geringer Anteil der Gallensäure im peripheren Blut. Die Konzentrationen der einzelnen Gallensäuren in Serum sind daher in der Regel niedrig. Dort sind sie an Albumin und Lipoproteine, die die Transportfunktion übernehmen, gebunden. Die Stärke der Gallensäurebindung nimmt mit dem hydrophoben Charakter der Gallensäure zu (G. Salvioli et al., Bile Acid Binding in Plasma: the importance of lipoproteins. FEBS-Letters 187, 272-276 (1985)). Die Konzentration der Gallensäuren im Serum wird beeinflußt durch die Gallensäuresynthese, ihre Sekretion und Rückresorption im Darm und durch erneute Aufnahme in die Leber. Sind diese Funktionen gestört, kann es zu Veränderungen des Serumgallensäureprofils kommen, d.h., die Gesamtgallensäurekonzentration kann im Vergleich zum Gesunden erhöht oder erniedrigt sein. Es können sich aber auch die Konzentrationen nur einzelner Gallensäuren ändern, z.B. bei Behandlung mit Gallensäurepräparaten. So ist bei Lebererkrankungen die Konzentration der Gallensäuren im Serum meist erhöht und das Verhältnis der endogenen Gallensäuren zueinander oft stark verändert (C.R. Pennington et al. Serum Bile Acids in the Diagnosis of Hepatobiliary Disease. Gut 18, 903-908,1977).

Die Bestimmung der Gallensäuren kann mit Hilfe spektroskopischer Methoden (S.J. Levin et al. Spectrofluorometric determination of total bile acids in bile. Anal. Chem. 33, 856-860, 1961), Radioimmunoassays (J.D. Palombo et al. Assessment of the effects of above-normal bilirubin on radioimmunoassay of

conjugated bile acids in serum. Clin Chem. 32, 2204-2205,1986), Dünnschichtchromatographie (S.S. Ali et al. Quantitative estimation of bile salts in Serum. Can. J. Biochem 48, 1054-1057, 1970), HPLC (G.R. Cambell et al. Modified sample preparation and chromatography for the separation of human bile acid conjugates. Anal. Proceed. 23,33-34, 1986) oder Gaschromatographie (T. Laatikainen et al. Determination of serum bile acids by glass capillary gas-liquid chromatography. Clin. Chim. Acta 64, 63-68, 1975) erfolgen. HPLC und Gaschromatographie stellen aufgrund hoher Trennleistung gute Bestimmungsmethoden dar, jedoch kann bei gaschromatographischen Methoden nicht auf eine Derivatisierung verzichtet werden, z.B. Silylierung der OH-Gruppen und Veresterung bzw. Methylierung der Carboxylfunktion. Daher erfordert die GC wesentlich höheren Zeitaufwand und auch eine hohe Reinheit der Proben, was wiederum einen aufwendigen Probenvorbereitungsprozess erforderlich macht.

Die bislang am häufigsten angewandte Probenvorbereitungsmethode von K.D.R. Setchell et al. (A rapid method for the quantitative extraction of bile acids and their conjugates from serum using commercially available reversed-phase octadecylsilane bonded silica cartridges. Clin. Chim. Acta 125, 135-144, 1982) erweist sich in der Praxis als nicht reproduzierbar und hat den Nachteil, daß große Probenvolumina benötigt werden, um ausreichende Mengen zur weiteren Analyse anzureichern (R. Nuber et al. J. Lipid Research 31, 1517-1522, 1990).

Nun hat wiederum die Methode von Nuber et al. den Nachteil, daß ein hoher Probendurchsatz durch die aufwendige Vorbehandlung der Serumproben unmöglich ist und sich somit nur für ausgewählte Proben empfiehlt.

Auf der Suche nach einer Alternative zu den bislang beschriebenen Methoden wurde überraschenderweise gefunden, daß sich die Verbindung nach Beispiel besonders gut zur selektiven und quantitativen Adsorption von Gallensäuren in Serum eignet und eine ebenfalls quantitative Desorption erlaubt, so daß es möglich wird, die Gallensäuren effizient von ihren Serum-Carrier-Proteinen zu trennen, nach Desorption zu derivatisieren und einer hochempfindlichen Fluoreszenz-HPLC-Methodezuzuführen.

## Beispiel

4,3 g (0,1 mol) Polyethylenimin (in 50 % wäßriger Lösung) werden mit 100 ml Wasser verdünnt und mit 23,7 g (0,2 mol) Chlorcyclohexan 24 h unter starkem Rühren zum Rückfluß erhitzt. Nach kurzer Abkühlung gibt man 100 ml 2N NaOH hinzu und erhitzt erneut 24 h zum Rückfluß.

Die organische Phase wird abgetrennt. Die wäßrige Phase wird mit Dichlormethan gewaschen und am Rotationsverdampfer und danach im Ölpumpenvakuum eingeengt. Der Rückstand wird aus wäßriger Lösung dialysiert.

Nach Entfernen des Wassers (Rotationsverdampfer, Ölpumpenvakuum, Gefriertrocknung) erhält man das mit Cyclohexan substituierte Polyethylenimin als schwach gelbliches Pulver (Charakterisierung durch Elementaranalyse).

Die erfindungsgemäße Anwendung in einem analytischen Verfahren zeigt der folgende Verfahrensablauf:

Analytisches Verfahren zur Anreicherung von Gallensäuren aus Serum unter Verwendung cycloalkylierter Polyethylenimine:

1. Schritt: Deproteinierung
   - 1 ml Serum + 100µl NaOH (1M) 20 Min bei 96° C inkubieren.
   - 100 µl HCl (2M) zugeben, mischen (ergibt pH-Wert zwischen 3 und 4), abzentrifugieren.
   - Pellet in 500 µl NaOH s.o. lösen, 20 Min bei 96° C inkubieren.
   - 300 µl HCl s.o. zugeben, abzentrifugieren.
2. Schritt: Gallensäurebindung
   - Die beiden Überstände aus Schritt 1 gemeinsam in ein Eppendorfgefäß mit 150 mg Verbindung nach Beispiel geben.
   - 4 Stunden bei 37° C im Eppendorfschüttelheizer inkubieren.
   - Abzentrifugieren, Überstand verwerfen.
3. Schritt: Adsorber entfernen
   - 600 µl NaOH 1M zugeben, 20 Min bei 96° C im Eppendorfheizer inkubieren (Adsorber löst sich auf).
   - 300 µl methanolische HCl ca. 5M zugeben, um Adsorber und Proteinreste auszufällen.
   - Abzentrifugieren, Überstand sammeln.
   - In 600 µl NaOH s.o. lösen, 20 Min bei 96° C inkubieren.
   - 300 µl meth. HCl s.o. zugeben, abzentrifugieren.
4. Schritt: Einengen

- Überstände eindampfen, in 0,5 ml MeOH aufnehmen. Dazu die Probe einige Zeit im Eppendorfschüttler schütteln.

5. Schritt: Solvolyse (Princen HMG., Meijer P. and Kuipers, F. One-Step solvolysis of 3-, 7- and 12-sulfated free and conjugated bile acids. Clin. Chim. Acty 192, 77-84, 1990)

- Probe aus Schritt 4 in 5 ml Dioxan-HCl geben, in Reagenzgläsern über Nacht bei 37°C im Schüttelwasserbad inkubieren.
- Eindampfen, in 0,5 ml MeOH wieder aufnehmen.

6. Schritt: Derivatisierung für HPLC mit Fluoreszenzdetektion

- gesamte Probe aus Schritt 5 einsetzen, mit 200 µl methanolischer KOH eindampfen.
- in 100 µl Dicyclohexano-18-Kronenether (1 mg/ml Acetonitril) aufnehmen, 100 µl 4-Bromomethyl-6,7-dimethoxycoumarin (1,5 mg/ml Acetonitril) zugeben.
- 40 Min bei 37°C im Wasserbad inkubieren.
- 100 µl Acetonitril zugeben, über Nacht im Tiefkühlschrank einfrieren.
- Analyse in der HPLC.

Die Derivatisierung. HPLC mit Fluoreszenzdetektion (unkonjugierte und glycinkonjugierte Gallensäuren), erfolgt wie unten angegeben:

| | |
|---|---|
| Geräte: | HPLC-Anlage der Fa. Kontron, bestehend aus drei Pumpen und Mischkammer, Autosampler, UV-Detektor und Auswerteeinheit mit Software MT2. Fluoreszenzdetektor von Merck-Hitachi. Da die Proben licht- und temperaturempfindlich sind, wird der Autosampler auf ca. 5°C gekühlt. |
| Mobile-Phase: | Laufmittel A: ®Millipore-Wasser (eigene Anlage) Laufmittel B: Acetonitril/Methanol 60:30 |
| Säule: | ®LiChrospher 100 RP-18, 25 mm, 5 µm von Merck |
| Vorsäule: | LiChrospher 60 RP-select B, 4 mm, 5 µm von Merck |
| Flußrate: | 1,3 ml/min |
| Detektion: | |

| | |
|---|---|
| Excitation: | 340 nm |
| Emission: | 410 nm |

Gradient:

| | |
|---|---|
| 0,00 min | 66 % B |
| 7,50 min | 66 % B |
| 8,00 min | 76 % B |
| 12,50 min | 76 % B |
| 13,00 min | 83 % B |
| 25,00 min | 83 % B |
| 25,50 min | 91 % B |
| 40,00 min | 91 % B |

Die folgende Tabelle 1 zeigt die mit den erfindungsgemäß verwendeten Verbindungen erreichbaren Anreicherungsraten [%]:

Tabelle 1

Bestimmung der Recovery Rate mit Hilfe von radioaktiv markierten Gallensäuren (Taurocholat TC, Glycocholat GC und Cholat C)

| | | |
|---|---|---|
| | TC: | 105 % |
| Deproteinierung | GC: | 106 % |
| | C: | 102 % |
| | TC: | 79 % |
| Gallensäurebindung | GC: | 78 % |
| | C: | 88 % |
| | TC: | 87 % |
| Adsorber entfernen | GC: | 99 % |
| | C: | 73 % |
| | TC: | 43 % |
| Einengen | GC: | 66 % |
| | C: | 74 % |
| | TC: | 92 % |
| Solvolyse | GC: | 77 % |
| | C: | 53 % |

## Patentansprüche

1. Verwendung unvernetzter alkylierter Polyethylenimine herstellbar aus einem Ausgangspolyethylenimin mit einem Molekulargewicht von 10 000 bis 10 000 000 und einem Alkylierungsmittel, dadurch gekennzeichnet, daß das Alkylierungsmittel die allgemeine Formel I besitzt

   R-X (I)

   worin
   - R ein Cycloalkylrest mit 5-30 Kohlenstoffatomen bedeutet und
   - X Chlor, Brom, Jod, $CH_3-SO_2-O-$ oder

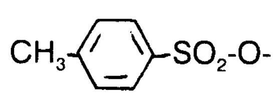

   bedeutet, zur Anreicherung von Gallensäuren und deren Derivaten aus biologischen Flüssigkeiten oder

Extrakten.

2. Verwendung von Polyethyleniminen nach Anspruch 1, dadurch gekennzeichnet, daß der Cycloalkylrest mono-, bi-, tricyclisch oder polycyclisch ist und/oder verbrückt ist.

3. Verwendung von Polyethyleniminen nach den Ansprüchen 1 oder 2, dadurch gekennzeichnet, daß R Cylopentyl, Cyclohexyl, Cycloheptyl, Cyclooctyl, Cyclononyl, Cyclodecyl, Decalyl, Hydrindanyl, Norbornyl oder Cyclopentanoperhydrophenanthren und deren Derivate ist.

4. Verwendung von Polyethyleniminen nach Anspruch 1, dadurch gekennzeichnet, daß das Ausgangspolyethylenimin ein Molekulargewicht von größer als 100 000 aufweist.

5. Verwendung von Polyethyleniminen nach Anspruch 1, dadurch gekennzeichnet, daß der Cycloalkylrest mono-, bi-, tricyclisch oder polycyclisch ist und/oder verbrückt ist, und daß das Ausgangspolyethylenimin ein Molekulargewicht von größer als 100 000 aufweist.

6. Verwendung von Polyethyleniminen nach Anspruch 1, dadurch gekennzeichnet, daß das Ausgangspolyethylenimin ein Molekulargewicht von größer 100 000 aufweist, und daß R Cyclopentyl, Cyclohexyl, Cycloheptyl, Cyclooctyl, Cyclononyl, Cyclodecyl, Decalyl, Hydrindanyl, Norbornyl oder Cyclopentanoperhydrophenanthren und deren Derivate ist.

7. Analytisches Verfahren zur Anreicherung von Gallensäuren und deren Derivaten an einer Festphase, enthaltend unvernetzte alkylierte Polyethylenimine der Ansprüche 1 bis 6, bestehend aus folgenden Schritten:
   - Deproteinierung des Serums und des Adsorbers,
   - Zusammenbringen des Serums und des Adsorbers,
   - Entfernen des Adsorbers,
   - Einengen des Überstandes,
   - Solvolyse,
   - Derivatisierung der Probe.

8. Verfahren nach Anspruch 7, dadurch gekennzeichnet, daß das Serum Human- oder Animal Serum ist.

9. Verfahren nach den Ansprüchen 7 und 8, dadurch gekennzeichnet, daß es sich um eine Ionenaustauschchromatographie handelt.

Europäisches Patentamt

# EUROPÄISCHER RECHERCHENBERICHT

Nummer der Anmeldung

EP 92 11 5925

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,Y | EP-A-0 379 161 (HOECHST)<br>* das ganze Dokument *<br>--- | 1-9 | A61K31/785<br>C08G73/04<br>B01J20/32 |
| Y | US-A-3 383 281 (F. J. WOLF)<br>* Spalte 2, Zeile 16 - Zeile 20 *<br>* Spalte 3, Zeile 1 - Zeile 8 *<br>* Spalte 3, Zeile 40 - Zeile 48 *<br>* Ansprüche *<br>--- | 1-9 | |
| Y | EP-A-0 143 423 (J. T. BAKER)<br>* Ansprüche *<br>* Seite 1, Zeile 11 - Zeile 15 *<br>* Seite 8, Zeile 7 - Zeile 15 *<br>----- | 1-9 | |
| | | | RECHERCHIERTE SACHGEBIETE (Int. Cl.5 ) |
| | | | A61K<br>C08G<br>B01J |

Der vorliegende Recherchenbericht wurde für alle Patentansprüche erstellt

| Recherchenort | Abschlußdatum der Recherche | Prüfer |
|---|---|---|
| DEN HAAG | 08 DEZEMBER 1992 | SCARPONI U. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)